(19) Europäisches Patentamt
European Patent Office
Office européen des brevets

(11) Publication number: **0 141 876 A1**

# (12) EUROPEAN PATENT APPLICATION

(21) Application number: **83111440.0**

(22) Date of filing: **15.11.83**

(51) Int. Cl.[4]: **C 07 C 51/10**, C 07 C 59/205

(43) Date of publication of application: **22.05.85**
**Bulletin 85/21**

(84) Designated Contracting States: **AT BE CH DE FR GB IT LI LU NL SE**

(71) Applicant: **ETHYL CORPORATION, Ethyl Tower 451 Florida Boulevard, Baton Rouge Louisiana 70801 (US)**

(72) Inventor: **Lee, John Yuchu, 1524 Stoneleigh Drive, Baton Rouge Louisiana 70809 (US)**
Inventor: **Walter, Thomas James, 10686 Danbury Drive, Baton Rouge Louisiana 70809 (US)**

(74) Representative: **Berg, Wilhelm, Dr. et al, Patentanwälte Dr. Berg Dipl.-Ing. Stapf Dipl.-Ing. Schwabe Dr. Dr. Sandmair Postfach 86 02 45 Stuntzstrasse 16, D-8000 München 86 (DE)**

## (54) Preparation of cyclic-keto-acids.

(57) Cyclic-keto-acids of the general formula:

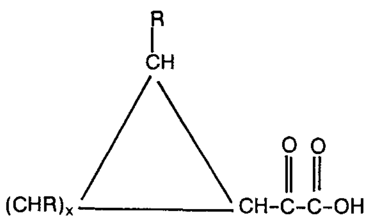

wherein R is hydrogen or a monovalent alkyl radical which can be either straight chain or branched having from 1 to 20 carbon atoms and X = 1, 3, 4 or 5, are prepared by reacting a functionalized 1-bromoalkane of the formula:

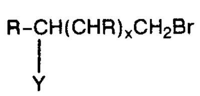

wherein R and X are as defined above and Y is a leaving group, preferably the halogens, in a liquid solvent medium with carbon monoxide at elevated temperature and pressure in the presence of a catalytic amount of a metal carbonyl compound and an alkali or an alkaline earth metal inorganic base.

EP 0 141 876 A1

0141876



Case 4925

# PREPARATION OF CYCLIC-KETO-ACIDS

The present invention relates to a process for the carbonylation of a functionalized 1-bromoalkane to form a cyclic-keto-carboxylic acid as the predominant product.

The practical value of such acids and their esters is that they can be used in the synthesis of pharmaceuticals, such as steroids, and for preparing herbicides.

The preparation of α-keto-carboxylic acids and their derivatives has been the subject of a large number of investigations. According to Rodd, The Chemistry of Carbon Compounds (1952 edition), Vol. 1, pages 227-229, the following methods of preparation are available:

- gentle oxidation of α-hydroxyacids containing a secondary hydroxyl group, or by the enzymatic deamination of α-amino-acids;
- hydrolysis of an acyl cyanide;
- hydrolysis of α-oximino-esters;
- from glycidic acid esters on treatment with benzene saturated with boron trifluoride;
- from α,β-dibromocarboxylic acids by forming a piperidine addition compound followed by hydrolysis;

from α-keto-acetals by ultraviolet irradiation in the presence of N-bromosuccinimide;

from α-bromomethylketones by boiling with selenium dioxide in absolute methanol or ethanol;

from carboxylic acid esters by oxidation with selenium dioxide;

permanganate oxidation of vinyl ketones;

from carboxylic acid esters by condensation with oxalic ester followed by decarboxylation;

from aldehydes via 5-alkylidene-2-thio-oxazolid-4-ones or by reaction with methyl methoxyacetate;

hydrolysis of azlactones or acetamido-acrylic acids;

hydrolysis of the reaction product of Grignard reagents on diethyl-oxamic ester;

oxidation of α-hydroxyacid esters containing two α-hydrogen atoms by N-bromosuccinimide in carbon tetrachloride to β-bromo-α-keto-acid esters; and

by the action of alkali on the dimethane-sulphonates and ditoluene-*p*-sulphonates of α, β-dihydroxy-carboxylic acids.

Methods for preparing arylpyruvic acids also are known. For example, U. S. Pat. No. 4,152,352 discloses

the preparation of an arylpyruvic acid by reacting an arylmethyl halide in a liquid solvent medium with carbon monoxide at pressures of 5 to 200 bars in the presence of a catalytic amount of a metal carbonyl compound and an alkaline earth metal inorganic base. Further, U. K. Patent Application 2,026,478A discloses that alkali metal salts of an arylpyruvic acid can be prepared by reacting an arylmethyl halide, carbon monoxide and an alkali metal base in the presence of a metal carbonyl compound as catalyst and in the presence of an alcohol or cyclic ether as solvent.

The cobalt-catalyzed carbonylation of secondary benzyl halides to give either monocarbonyl or double carbonyl insertion or coupling of organic halides is reported by E. Francalanci et. al., Journal of Electroanalytical Chemistry, 1982, pp. 59-70.

It has now been found that cyclic-keto-acids of the general formula:

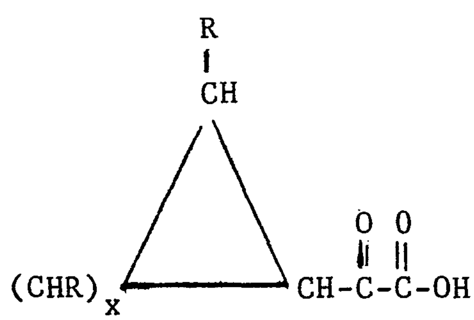

in which R represents hydrogen or a monovalent alkyl radical which can be either straight chain or branched containing from 1 to 20 carbon atoms, and X = 1, 3, 4 or 5 can be prepared by carbonylating a functionalized 1-bromoalkane of the general formula:

$$\underset{\displaystyle Y}{\underset{|}{R\text{-}CH}}(CHR)_xCH_2Br$$

where R and X are as defined above and Y is a leaving group, preferably a halogen, in a liquid solvent medium, with carbon monoxide at a pressure of from 300 to 3000 psig in the presence of a catalytic amount of a metal carbonyl compound and an alkali or an alkaline earth metal inorganic base.

The functionalized 1-bromoalkane reactants suitable for use in the present process are well known in the art as are methods for their preparation and, as defined above, are of the general formula:

$$\underset{\displaystyle Y}{\underset{|}{R\text{-}CH}}(CHR)_xCH_2Br$$

when R is hydrogen or a monovalent alkyl radical which can be either straight chain or branched containing from 1 to about 20 carbon atoms, X = 1, 3, 4 or 5 and Y is a

suitable leaving group,preferably the halogens viz., iodine, bromine and most preferably chlorine. Other suitable leaving groups, such as, for example, nitrates, sulfonates and acetates also may be used with the proviso that under the reaction conditions of the instant process, hydrolysis does not become a major competing reaction with the resultant production of unwanted alcohols as the major product. A few exemplary materials of this type include 1-bromo-3-chloro-or 3-bromopropane, butane, pentane, hexane, heptane, octane, nonane, decane, undecane, dodecane, tridecane, tetradecane, pentadecane, hexadecane, heptadecane, octadecane, nonadecane, eicosane, and the like; 1-bromo-5-chloro- or 5-bromo- pentane, hexane, heptane, octane, nonane, decane, undecane, dodecane, tridecane, tetradecane, pentadecane, hexadecane, heptadecane, octadecane, nonadecane, eicosane, and the like; 1-bromo-6-chloro- or 6-bromohexane, heptane, octane, nonane, decane, undecane, dodecane, tridecane, tetradecane, pentadecane, hexadecane, heptadecane, octadecane, nonadecane, eicosane and the like; 1-bromo-7-chloro- or 7-bromoheptane, octane, nonane, decane, undecane, dodecane, tridecane, tetradecane, pentadecane, hexadecane, heptadecane, octadecane, nonadecane, eicosane, and the like; the nitrate,

sulfonate and acetate esters of 1-bromo-3-hydroxypropane, butane, pentane, hexane, heptane, octane, nonane, decane, undecane, dodecane, tridecane, tetra-decane, pentadecane, hexadecane, heptadecane, octa-decane, nonadecane, eicosane and the like; the nitrate, sulfonate and acetate esters of 1-bromo-5-hydroxy-pentane, hexane, heptane, octane, nonane, decane, undecane, dodecane, tridecane, tetradecane, pentadecane, hexadecane, heptadecane, octadecane, nonadecane, eicosane and so forth. A particularly useful reactant is 1-bromo-3-chloropropane from which cyclopropylglyoxylic acid (a precursor for the synthesis of steroids) is produced by the process of the present invention.

The reaction is carried out in the presence of a mixture of water and alcohol as a reaction medium. Preferably, the alcohols employed for the reaction may be straight-chain, branched or cyclic, and preferably contain up to 6 carbon atoms. Methanol, ethanol, propanol, isopropanol, n-butanol, isobutanol, tert-butanol, and tert-amyl alcohol may be mentioned as examples. Cyclic ethers, such as tetrahydrofuran, also may be used. A particularly preferred solvent alcohol is tert-butanol. Mixtures containing 10% to 90% by weight of water and 90% to 10% by weight of alcohol

generally are used. Preferred mixtures contain 30% to 80% by weight water and 70% to 20% by weight alcohol.

The reaction takes place in the presence of a basic substance suitably an alkali or an alkaline earth metal hydroxide employing a metal carbonyl compound. During the reaction, the 1-bromoalkane undergoes reaction with the carbon monoxide and basic substance whereby the salt of the desired cyclic-keto-carboxylic acid is formed from which the cyclic-keto-carboxylic acid is isolated after acidification in a known manner. It is believed that the salt of the keto acid is formed first followed by intramolecular nucleophilic displacement under the alkaline conditions of the reaction medium to form the cyclic keto acid.

Specific examples of suitable basic agents which can be used in the practice of the process include: LiOH, NaOH, KOH, RbOH, $Ca(OH)_2$, $Ba(OH)_2$ and $Mg(OH)_2$. LiOH and $Ca(OH)_2$ are particularly preferred. Yields of cyclic-keto-carboxylic acids of up to approximately 62% can be obtained using $Ca(OH)_2$ as the basic substance and a solvent medium of tert-butanol and water.

The amount of basic agent used can vary within wide limits. In general, the molar ratio of the alkali metal or alkaline earth metal base to 1-bromoalkane reactant is preferably 10:1 to 1:1.

In the process described herein, it is preferred to use metal carbonyl compounds as carbonylation catalysts. These catalysts include particularly metal carbonyls such as iron pentacarbonyl, dicobalt-octacarbonyl and nickel-tetracarbonyl, or their salts such as, for example, the calcium, potassium or sodium salts thereof. Dicobalt-octacarbonyl is very particularly suited. These catalysts can be added to the medium in the solid state or in the form of solutions in the solvent used for the carbonylation reaction. The molar percentage of the metal carbonyl compound to the 1-bromoalkane reactant is preferably from 0.1 to 25%.

The concentration of the 1-bromoalkane used in the reaction solvent is not critical and can vary within wide limits. Thus, it can be between 1 and 30% by weight, based on the weight of the solvent, however, it is possible to go outside of these limits without disadvantage.

The present process is advantageously carried out by bringing the mixture consisting of the 1-bromoalkane reactant, the metal carbonyl catalyst and the alkali or alkaline earth metal base, suspended in the mixture of water and alcohol, into contact, under nitrogen, in a suitable pressure-resistant reactor equipped with a stirrer, with a large excess of carbon monoxide (amount greater than 2 moles of carbon monoxide per mole of the

starting 1-bromoalkane reactant) introduced at the desired pressure and temperature, in accordance with techniques suitable for bringing about the reaction between a liquid phase and a gas phase.

The carbonylation reaction is carried out at a temperature in the range of from 30°C. to 150°C., preferably from 50°C. to 100°C., over a period of time of from 3 to 60 hours, typically 3 to 20 hours.

In general, the reaction takes place at elevated carbon monoxide pressures which may range from 300 psig to 3000 psig. Preferably, the reaction takes place at a pressure in the range of 500 psig to 1000 psig. The carbon monoxide may contain or be mixed with an inert gas, such as nitrogen.

On completion of the reaction, the product mixture is filtered, resulting in the alkali metal or alkaline earth metal salt of the cyclic-keto-carboxylic acid being separated from the liquid reaction components as the main solid component. The filtrate contains the remainder of the alkali or alkaline earth metal salt of the cyclic-keto-carboxylic acid, and, where unbranched alcohols are used, also esters in addition to unreacted 1-bromoalkane as well as acid and alcohol products from the starting 1-bromoalkane.

In a further process step, the metal salt of the cyclic-keto-carboxylic acid is acidified with a dilute

acid, such as hydrochloric acid, so as to displace the cyclic-keto-carboxylic acid from its alkali or alkaline earth metal salt.

If desired, lower alkyl esters of the cyclic-keto-carboxylic acid products of the present invention can be prepared by esterifying the cyclic-keto-carboxylic acid product according to conventional esterification techniques employing lower aliphatic alkanol and acid catalysts such as, for example, $BF_3$, $BF_3$ HCl, or $BF_3$ MeOH, $BF_3$ $Et_2O$ or diazomethane at suitable reaction conditions.

The following example illustrates the invention.

Example 1

Into a 300 ml autoclave were charged 7.87 g (50 mmoles) of 1-chloro-3-bromopropane and 70 ml of t-BuOH. Next, 0.682 g (2 mmoles) of $Co_2(CO)_8$ were added under CO, and then a mixture of 7.4 g (100 mmoles) of lime and 30 ml of $H_2O$ were added. After 850 psi CO was charged to the autoclave, the reaction mixture was heated to 80$^oC$ over a period of time of approximately 1 hour and held at that temperature for 15 hours. The CO uptake stopped after 11 hours. After centrifugation, the solid was rinsed once with a 20 ml portion of a 50:50 t-butanol/water solution and then acidified with 150 ml of HCl solution containing 100 mmoles of HCl. The free acid was extracted from the aqueous solution

with diethyl ether (2 x 120 ml) to give a 62% yield of cyclopropylglyoxylic acid based on proton NMR data with internal standard.

CLAIMS:

1. A process for the production of cyclic-keto-acids of the general formula:

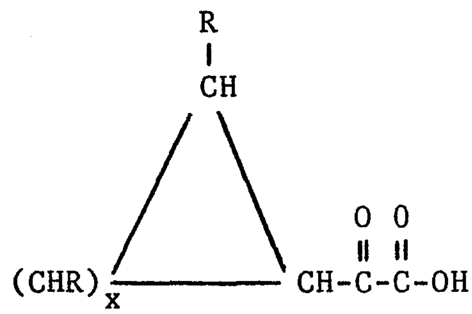

where R is hydrogen or a monovalent alkyl radical which can be either straight chain or branched having from 1 to 20 carbon atoms and X = 1, 3, 4 or 5 which comprises reacting a functionalized 1-bromoalkane of the formula:

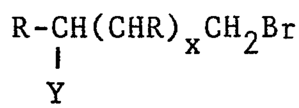

where R and X are as defined above and Y represents a leaving group, in a liquid solvent medium, with carbon monoxide at elevated temperature and pressure in the presence of a catalytic amount of a metal carbonyl compound and an alkali or an alkaline earth metal inorganic base.

2. The process as claimed in Claim 1 in which the functionalized 1-bromoalkane is 1-bromo-3-chloro- or 3-bromopropane.

3. The process as claimed in Claim 1 in which the leaving group is selected from the halogens, nitrates, sulfonates or acetates.

4 The process as claimed in Claim 1 in which the carbon monoxide pressure is from 300 to 3000 psig.

5. The process as claimed in Claim 1 in which the reaction is carried out at a temperature of from 30°C. to 150°C.

6. The process as claimed in Claim 1 in which the inorganic base is selected from LiOH, NaOH, KOH, RbOH, $Ca(OH)_2$, $Ba(OH)_2$ or $Mg(OH)_2$.

7. The process as claimed in Claim 1 in which the metal carbonyl is dicobalt-octacarbonyl.

8. The process as claimed in Claim 1 in which the liquid solvent medium is a mixture of water and alcohol.

9. The process as claimed in Claim 8 in which the alcohol is tert-butanol.

10. The process as claimed in Claim 8 in which the alcohol is isopropanol.

European Patent Office

# EUROPEAN SEARCH REPORT

0141876

Application number

EP 83 11 1440

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-1 533 818 (DEGUSSA) * Claims 1,6 * ----- | 1 | C 07 C 51/10<br>C 07 C 59/205 |
| | | | TECHNICAL FIELDS SEARCHED (Int. Cl. 3) |
| | | | C 07 C 51/00<br>C 07 C 59/00 |

The present search report has been drawn up for all claims

| Place of search | Date of completion of the search | Examiner |
|---|---|---|
| THE HAGUE | 10-07-1984 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document